# EUROPÄISCHE PATENTSCHRIFT

(11) **EP 2 249 139 B1**
(45) Veröffentlichungstag und Bekanntmachung des Hinweises auf die Patenterteilung: **19.08.2015**
(21) Anmeldenummer: 10002587.3
(22) Anmeldetag: 11.03.2010
(51) Int. Cl.: G01N 1/06, A61F 11/00, A61B 17/00, A61B 19/00

(54) **Einweg-Knorpelschneider**
Disposable cartilage cutter
Dispositif de coupe de cartilage à usage unique

(30) Priorität: 06.05.2009 DE 202009006583 U
(43) Veröffentlichungstag der Anmeldung: 10.11.2010
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus Patentanwälte

(56) Entgegenhaltungen:
- EP-B1- 0 483 567
- DE-U1- 20 012 662
- US-A- 4 288 066

## Beschreibung

Die Erfindung betrifft eine medizinische Schneidevorrichtung zur Herstellung dünner Knorpelscheiben mit einem Vorrichtungskörper und einem Deckel, wobei eine erste Halteeinrichtung vorgesehen ist, die einen ersten Abschnitt mit einer auf der Oberseite des Vorrichtungskörpers angeordneten ersten Vertiefung aufweist, welche von einem ersten Begrenzungssteg ganz oder teilweise umrandet und durch einen auf der Oberseite des Deckels angeordneten ersten Vorsprung verschließbar ist, und wobei der erste seitliche Begrenzungssteg einen von einer Stirnseite des ersten Abschnitts her geführten, mit einem vorgegebenen ersten Abstand parallel zur Grundfläche der ersten Vertiefung verlaufenden ersten Führungsschlitz zum Einschieben einer Schneidklinge aufweist.

Eine derartige Vorrichtung ist bekannt aus der EP 0 483 567 B1.

Das Herausschneiden von dünnen körpereigenen Knorpelscheiben gleichmäßiger Dicken aus einem größeren, z.B. aus der Ohrmuschel, dem Tragus, dem knorpeligen Anteil der oberen Rippenknochen oder dem Nasenseptum entnommenen Knorpelstück ist für verschiedene medizinische Zwecke eine immer wieder erforderliche Tätigkeit. Beispielsweise kann es notwendig sein, spezielle Eigenschaften des Grundkörpers näher zu untersuchen, insbesondere unter einem Mikroskop. In der Hals-Nasen-Ohren-Heilkunde werden solche dünnen Knorpelscheiben aber auch bei vielen chirurgischen Eingriffen benötigt, etwa im Mittelohr-Bereich zum Abdecken einer Mittelohr-Prothese, zum Wiederaufbau der Gehörgangshinterwand oder zur plastischen Versorgung eines Trommelfell-Defekts. Auch bei vielen nasenchirurgischen Eingriffen werden derartige dünne Knorpelscheiben eingesetzt, um funktionelle oder ästhetische Korrekturen an der Nase durchzuführen.

In der EP 0 483 567 B1 ist eine gattungsgemäße Schneidevorrichtung beschrieben, mit welcher dünne Knorpelscheiben einer - in gewissen Grenzen - vorgebbaren Dicke aus einem größeren Knorpelstück rasch, sicher und in gleichmäßiger Qualität herausgeschnitten werden können. Um unterschiedliche Dicken der erzeugten Knorpelscheiben erzielen zu können, müssen allerdings spezielle Distanzblättchen bekannter Dicke in die Schneidevorrichtung eingelegt werden. Diese Distanzblättchen müssen - ebenso wie die Schneidevorrichtung selbst - streng gereinigt und steril gehalten und vor jeder Operation eigens entsprechend behandelt werden, was einerseits zeitaufwändig und andererseits fehleranfällig ist. Bedenkt man, dass sich in einer durchschnittlichen HNO-Klinik drei bis vier Operationssäle befinden und zu Spitzenzeiten an einem Tag fünfzehn bis zwanzig Patienten operiert werden, so kann es sein, dass an einem Tag bis zu fünfzehn mal ein Knorpelschneider bereit gestellt werden muss. Dies ist für die Sterilgutversorgung eine große logistische Herausforderung.

Außerdem ist die Handhabung der Distanzblättchen nicht ganz einfach. So ist es zum Beispiel wegen ihrer geringen Größe nur bedingt möglich, sie ausreichend und gut erkennbar zu beschriften, was aber die Grundvoraussetzung dafür ist, dass während der Operation genau das Distanzblättchen mit der jeweils erforderlichen Größe bereit liegt. Auch das korrekte Einlegen und Fixieren der ziemlich kleinen Distanzblättchen in die Schneidevorrichtung erfordert einige Geschicklichkeit.

Aufgabe der vorliegenden Erfindung ist es demgegenüber, eine gattungsgemäße medizinische Schneidevorrichtung der eingangs beschriebenen Art mit möglichst einfachen technischen Mitteln unaufwändig und kostengünstig dahin gehend zu verbessern, dass dünne Knorpelscheiben bestimmter unterschiedlicher Dicken in gleichmäßiger Qualität auch ohne die Verwendung der bekannten Distanzblättchen hergestellt werden können.

Erfindungsgemäß wird diese Aufgabe auf ebenso überraschend einfache wie wirkungsvolle Weise dadurch gelöst, dass mindestens eine zweite Halteeinrichtung vorgesehen ist, die einen zweiten Abschnitt mit einer auf der Oberseite des Vorrichtungskörpers angeordneten zweiten Vertiefung aufweist, welche von einem zweiten Begrenzungssteg ganz oder teilweise umrandet und durch einen auf der Oberseite des Deckels angeordneten zweiten Vorsprung verschließbar ist, wobei der zweite seitliche Begrenzungssteg einen von einer Stirnseite des zweiten Abschnitts her geführten, mit einem vorgegebenen zweiten Abstand parallel zur Grundfläche der zweiten Vertiefung verlaufenden zweiten Führungsschlitz zum Einschieben einer Schneidklinge aufweist.

Da die Abstände zwischen dem jeweiligen Führungsschlitz und der entsprechenden Grundfläche der jeweiligen Vertiefung bei unterschiedlichen Halteeinrichtungen verschieden gewählt werden können, lassen sich mit der erfindungsgemäßen Schneidevorrichtung mit den unterschiedlichen Halteeinrichtungen Knorpelscheiben bestimmter unterschiedlicher Dicken herstellen, ohne dass dafür die bekannten Distanzblättchen eingesetzt werden müssen.

Weiter hat sich gezeigt, dass eine Handhabung der neuen Schneidevorrichtung zwischen Daumen und Zeigfinger dem Operateur mehr Sicherheit beim eigentlichen Schneidevorgang gibt. Alle Teile können aufgrund ihrer Geometrie und ihres Designs sicher und kontrolliert zueinander bewegt werden.

Eine besonders einfache und kompakte Ausführungsform der erfindungsgemäßen Schneidevorrichtung zeichnet sich dadurch aus, dass eine dritte Halteeinrichtung vorgesehen ist, die einen dritten Abschnitt mit einer auf der Oberseite des Vorrichtungskörpers angeordneten dritten Vertiefung aufweist, welche von einem dritten Begrenzungssteg ganz oder teilweise umrandet und durch einen auf der Oberseite des Deckels angeordneten dritten Vorsprung verschließbar ist, wobei der dritte Begrenzungssteg einen von einer Stirnseite des dritten Abschnitts her geführten, mit einem vorgegebenen dritten Abstand parallel zur Grundfläche der dritten Vertiefung verlaufenden dritten Führungsschlitz zum Einschieben einer Schneidklinge aufweist, und dass die drei Halteeinrichtungen relativ zueinander vorzugsweise in Kreuzform angeordnet sind.

In den meisten Fällen ist es nämlich erfahrungsgemäß ausreichend, wenn für eine Mittelohroperation drei verschiedene Dicken der herzustellenden Knorpelscheiben zur Auswahl stehen, um eine optimale Anpassung an die individuellen Gegebenheiten des Patienten durchführen zu können. Falls gleichwohl eine feinere Differenzierung erfolgen soll, können auch mehrere dieser Ausführungsformen nebeneinander verwendet werden, wobei dann jede einzelne Schneidevorrichtung einen anderen Dickenbereich abdecken sollte und eine Feinauswahl aufgrund der angebotenen drei verschiedenen Dicken im ausgewählten Dickenbereich erfolgen kann.

Ganz besonders bevorzugt ist Klasse von Ausführungsformen der erfindungsgemäßen Schneidevorrichtung, bei denen die Schneidevorrichtung aus einem sterilisierbaren Kunststoff hergestellt ist. Damit lässt sich die Schneidevorrichtung ganz erheblich preisgünstiger herstellen als die üblichen Vorrichtungen aus Metall. Die Anlieferung zur Operation erfolgt dann in einer Sterilverpackung und die benutzte Schneidevorrichtung kann einfach entsorgt werden. Ein solches steril verpacktes Einmal-Produkt hat außerdem den Vorteil, dass nicht vor jeder Operation eine aufwändige Reinigung und Sterilisation der Schneidevorrichtung erfolgen muss und es minimiert sich auch das Risiko einer Infektion, dass bei einer Sterilgutversorgung nicht ausgeschlossen werden kann.

Vorzugsweise wird bei Weiterbildungen dieser Klasse von Ausführungsformen die Schneidevorrichtung in einem Spritzgussverfahren hergestellt.

Bevorzugt sind auch Ausführungsformen der Erfindung, bei denen der Deckel über eine Materialbrücke am Vorrichtungskörper anhängt und vorzugsweise um 180° klappbar ist. Damit kann dann leicht eine Verschraubung der beiden Teile der Halteeinrichtungen gegeneinander erfolgen, wie dies an sich aus der EP 0 483 567 B1 bekannt ist um das Knorpelstück, aus welchem die gewünschte Knorpelscheibe herausgeschnitten werden soll, sicher zu fixieren.

Bei besonders einfach zu handhabenden Weiterbildungen dieser Ausführungsformen weist die Materialbrücke eine Soll-Biegelinie oder Verengung auf, um welche der Deckel gegenüber dem Vorrichtungskörper klappbar ist.

Ganz besonders bevorzugt ist eine Variante der oben beschriebenen Ausführungsformen, die sich dadurch auszeichnet, dass der Anspritzpunkt für das Spritzgussverfahren auf der Soll-Biegelinie angeordnet ist.

Eine weitere bevorzugte Klasse von Ausführungsformen der erfindungsgemäßen Schneidevorrichtung zeichnet sich dadurch aus, dass an den Abschnitten des Vorrichtungskörpers und/oder an den entsprechenden Abschnitten des Deckels, welche die Vorsprünge tragen, Kennzeichnungen angebracht sind, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

Bei Weiterbildungen dieser Ausführungsformen können die Kennzeichnungen Zahlen umfassen, die den jeweiligen vorgegebenen

Abstand des Führungsschlitzes zur Grundfläche der entsprechenden Vertiefung und somit die mit der jeweiligen Halteeinrichtung erzielbare Dicke der herzustellenden Knorpelscheibe im metrischen Maßsystem, insbesondere in Millimetern, oder im zöllischen Maßsystem, insbesondere in Inch, angeben.

Alternativ können die Kennzeichnungen aber auch graphische Darstellungen, insbesondere Skalenstriche, Punkte und dergleichen umfassen, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

Ergonomisch besonders günstig sind Varianten der oben beschriebenen Ausführungsformen, bei die Kennzeichnungen auf der den Vertiefungen gegenüberliegenden Unterseite des Vorrichtungskörpers und/oder auf der den Vorsprüngen gegenüberliegenden Unterseite des Deckels angebracht sind.

Bei einer weiteren ergonomisch günstigen Ausführungsform der erfindungsgemäßen Schneidevorrichtung sind auf der den Vertiefungen gegenüberliegenden Unterseite des Vorrichtungskörpers und/oder auf der den Vorsprüngen gegenüberliegenden Unterseite des Deckels konvexe und/oder konkave Griffhilfen angebracht, die eine Orientierungshilfe zur Ausübung eines Druckes auf das jeweilige Zentrum der Vorsprünge bieten.

Auf der den Vorsprüngen gegenüberliegenden Unterseite des Deckels kann bei Ausführungsformen der Erfindung eine randseitig umlaufende Umrandung vorgesehen sein.

Ebenso kann bei weiteren Ausführungsformen auf der Oberseite des Vorrichtungskörpers zwischen den Vertiefungen eine umlaufende Wandung vorgesehen sein, die einen Arbeitsraum umschließt, welcher beispielsweise zur Vorbearbeitung eines Knorpelstücks vor dem Abschneiden der gewünschten Scheibe oder zur weiteren Bearbeitung der abgeschnittenen Knorpelscheibe dienen kann.

Der Bearbeitung von abgeschnittenen Knorpelscheiben können auch runde und/oder ovale Templates unterschiedlichen Durchmessers dienen, welche bei Ausführungsformen der erfindungsgemäßen Schneidevorrichtung in einer Fläche der Schneidevorrichtung, insbesondere auf der die Vertiefungen tragenden Oberseite des Vorrichtungskörpers, vorzugsweise im Arbeitsraum, und/oder auf der die Vorsprünge tragenden Oberseite des Deckels eingearbeitet sind.

Ganz besonders vorteilhaft sind Ausführungsformen der erfindungsgemäßen Schneidevorrichtung, bei denen in einer Fläche der Schneidevorrichtung, insbesondere auf der die Vertiefungen tragenden Oberseite des Vorrichtungskörpers, vorzugsweise im Arbeitsraum, und/oder auf der die Vorsprünge tragenden Oberseite des Deckels eine Mess-Skala eingearbeitet ist, mit deren Hilfe die zu verarbeitenden Knorpelstücke und/oder die abgeschnittenen Knorpelscheiben problemlos vermessen werden können.

Um die zu verarbeitenden Knorpelstücke besser zu halten können bei Ausführungsformen der Erfindung die Grundflächen der Vertiefungen und/oder die Flächen der Vorsprünge aufgeraut sein.

Eine Klasse von Ausführungsformen zeichnet sich dadurch aus, dass die die Führungsschlitze tragenden seitlichen Begrenzungsstege jeweils einstückig umlaufend um die Vertiefungen gestaltet sind.

Bei einer dazu alternativen Klasse von Ausführungsformen sind die die Führungsschlitze tragenden seitlichen Begrenzungsstege jeweils als mehrere Einzelstege gestaltet, wie an sich aus der EP 0 483 567 B1 bekannt ist.

In der Praxis bewähren sich Ausführungsformen der erfindungsgemäßen Schneidevorrichtung, bei denen die Schneidklinge eine Messerklinge aus Metall ist, insbesondere eine Rasiermesser-Klinge, und die Schneidklinge in einer Messerhalterung gehalten wird, die vorzugsweise aus Kunststoff hergestellt ist.

Bei einfachen Weiterbildungen dieser Ausführungsformen kann die Messerhalterung einteilig ausgeführt sein und einen Schlitz zum Einschieben der Schneidklinge aufweisen.

In alternativen Weiterbildungen ist die Messerhalterung zweiteilig ausgeführt und zum Halten der Schneidklinge klappbar gestaltet, was ein Austauschen der Schneidklinge erleichtert.

Andere Weiterbildungen der oben beschriebenen Ausführungsformen sind dadurch gekennzeichnet, dass die Messerhalterung mindestens eine Fläche aufweist, in der runde und/oder ovale Templates, vorzugsweise unterschiedlichen Durchmessers, eingearbeitet sind.

Vorzugsweise ist eine randseitig um die Messerhalterung umlaufende Umrandung vorgesehen, welche das Flächeträgheitsmoment und damit die Stabilität der Halterung erhöht.

Eine andere Weiterbildung schließlich zeichnet sich dadurch aus, dass die Messerhalterung mindestens eine, vorzugsweise mehrere Durchgangsöffnungen aufweist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:

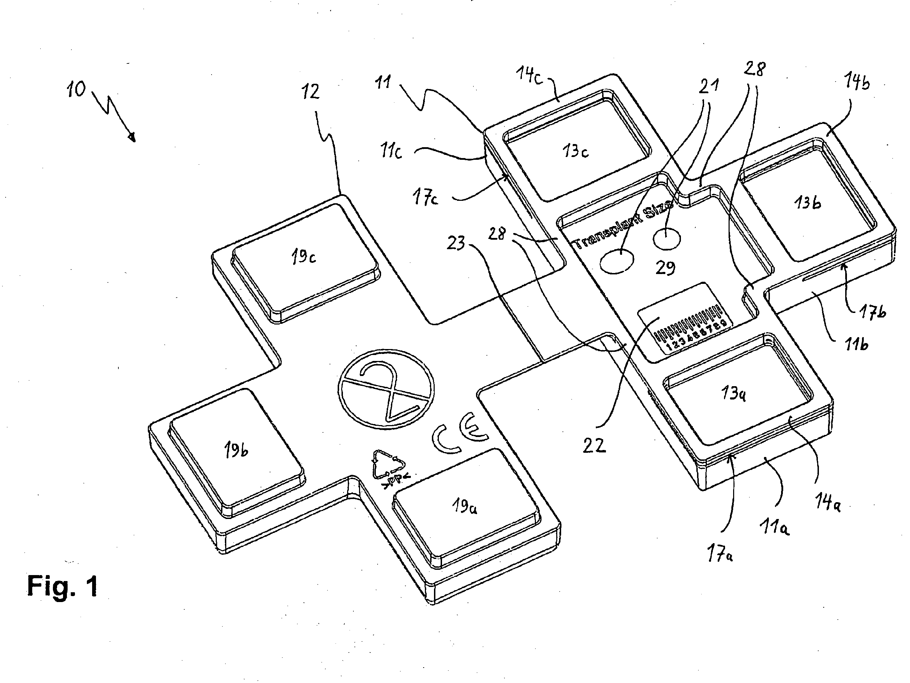

- Fig. 1: eine schematische räumliche Darstellung von der Oberseite einer Ausführungsform der erfindungsgemäßen Schneidevorrichtung mit drei kreuzförmig angeordneten Halteeinrichtungen;

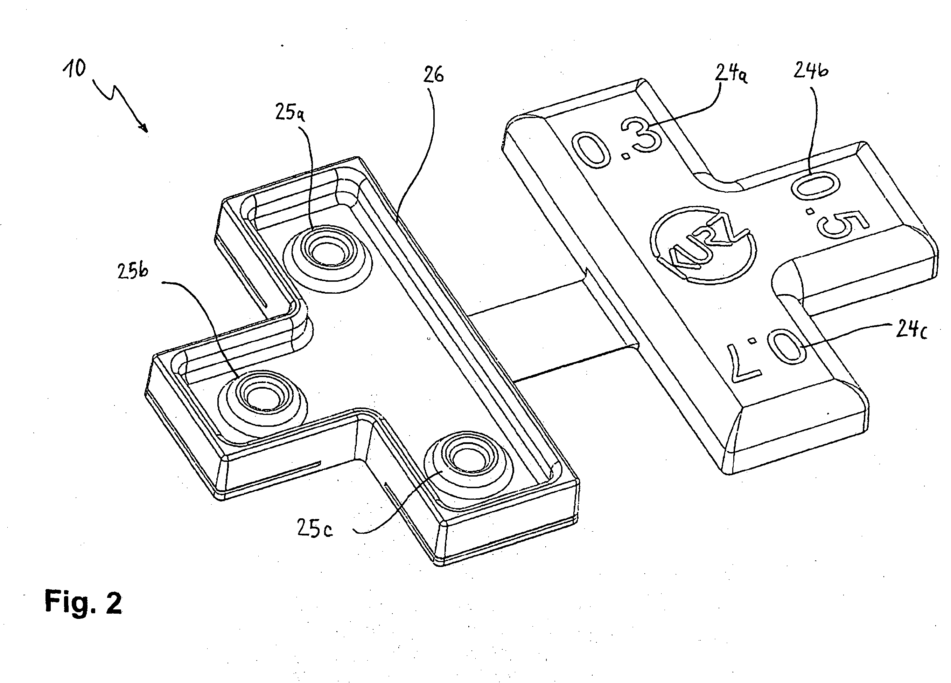

- Fig. 2: eine Ansicht auf die Unterseite der Ausführungsform aus Fig. 1;
- Fig. 3: eine Ansicht auf die Ausführungsform von Fig. 1 mit um eine Soll-Biegelinie geklapptem Deckel und in den Führungsschlitz einer der Halteeinrichtungen eingeschobener Schneidklinge mit Messerhalterung.

Die in den Figuren der Zeichnung schematisch räumlich dargestellte Ausführungsform der erfindungsgemäßen medizinischen **Schneidevorrichtung 10** dient zur Herstellung dünner Knorpelscheiben aus einem größeren Knorpelstück und ist aus einem sterilisierbaren Material, vorzugsweise aus sterilisierbarem Kunststoff und insbesondere in einem Spritzgussverfahren hergestellt. Sie umfasst einen **Vorrichtungskörper 11** und einen **Deckel 12.**

Außer einer ersten Halteeinrichtung zum Haltern des Knorpelstücks beim Zuschneiden einer Knorpelscheibe ist erfindungsgemäß noch mindestens eine zweite Halteeinrichtung und im vorliegenden Ausführungsbeispiel auch noch eine dritte Halteeinrichtung vorgesehen. Die drei Halteeinrichtungen sind bei der gezeigten Ausführungsform aus ergonomischen Gründen relativ zueinander in Kreuzform angeordnet.

Die Halteeinrichtungen umfassen jeweils einen **Abschnitt 11a, 11b, 11c** mit einer auf der Oberseite des Vorrichtungskörpers 11 angeordneten **Vertiefung 13a, 13b, 13c,** welche von einem einstückig ausgeführten **Begrenzungssteg 14a, 14b, 14c** ganz - sowie bei in der Zeichnung nicht dargestellten Ausführungsformen wahlweise auch jeweils durch mehrere Einzelstege nur teilweise - umrandet und durch einen auf der Oberseite des Deckels 12 angeordneten **Vorsprung 19a, 19b, 19c** verschließbar ist. Der seitliche Begrenzungssteg 14a, 14b, 14c weist einen von einer Stirnseite des Abschnitts 11a, 11b, 11c her geführten, mit einem vorgegebenen, je Halteeinrichtung unterschiedlichen Abstand parallel zur Grundfläche der Vertiefung 13a, 13b, 13c verlaufenden **Führungsschlitz 17a, 17b, 17c** zum Einschieben einer **Schneidklinge 18** auf. Die Grundflächen der Vertiefungen 13a, 13b, 13c und/oder die Flächen der Vorsprünge 19a, 19b, 19c sind für eine verbesserte Halterung der zu bearbeitenden Knorpelstücke vorzugsweise aufgeraut.

Der Deckel 12 hängt bei der dargestellten Ausführungsform der Erfindung über eine Materialbrücke mit einer **Soll-Biegelinie 23** am Vorrichtungskörper 11 an und ist um 180° gegenüber dem Vorrichtungskörper 11 klappbar. Der Anspritzpunkt für eine Herstellung der erfindungsgemäßen Schneidevorrichtung im Spritzgussverfahren kann auf der Soll-Biegelinie 23 angeordnet sein.

Weiter zeichnet sich die in der Zeichnung gezeigte Ausführungsform dadurch aus, dass an den Abschnitten 11a, 11b**,** 11c des Vorrichtungskörpers 11 **Kennzeichnungen 24a, 24b, 24c** in Form von Zahlenangaben angebracht sind, die den jeweiligen vorgegebenen Abstand des Führungsschlitzes 17a, 17b, 17c zur Grundfläche der entsprechenden Vertiefung 13a, 13b, 13c und somit die mit der jeweiligen Halteeinrichtung erzielbare Dicke der herzustellenden Knorpelscheibe - im vorliegenden Ausführungsbeispiel in Millimetern - angeben. Alternativ können die Kennzeichnungen die entsprechend Dickenmaßzahlen auch im zöllischen Maßsystem, insbesondere in Inch, angeben. Auch können die Kennzeichnungen alternativ oder ergänzend graphische Darstellungen, insbesondere Skalenstriche, Punkte und dergleichen umfassen.

In der Regel werden diese Kennzeichnungen 24a, 24b, 24c auf der den Vertiefungen 13a, 13b, 13c gegenüberliegenden Unterseite des Vorrichtungskörpers 11 und/oder auf der den Vorsprüngen 19a, 19b, 19c gegenüberliegenden Unterseite des Deckels 12 angebracht sein. Außerdem sind auf der den Vorsprüngen 19a, 19b, 19c gegenüberliegenden Unterseite des Deckels 12 - oder bei alternativen Ausführungsformen der Erfindung auf der den Vertiefungen 13a, 13b, 13c gegenüber liegenden Unterseite des Vorrichtungskörpers 11 - konvexe und/oder konkave **Griffhilfen 25a, 25b, 25c** sowie vorzugsweise auch eine randseitig umlaufende **Umrandung 26** angebracht.

Auf der Oberseite des Vorrichtungskörpers 11 zwischen den Vertiefungen 13a, 13b, 13c ist eine umlaufende **Wandung 28** vorgesehen, die einen **Arbeitsraum 29** umschließt.

In einer Fläche der Schneidevorrichtung 10 - im gezeigten Ausführungsbeispiel im Arbeitsraum 29 auf der die Vertiefungen 13a, 13b, 13c tragenden Oberseite des Vorrichtungskörpers 11 - sind runde sowie ovale **Templates 21** unterschiedlichen Durchmessers zur weiteren Bearbeitung der hergestellten Knorpelscheiben sowie eine **Mess-Skala 22** zur Vermessung der bearbeiteten Knorpelscheiben eingearbeitet.

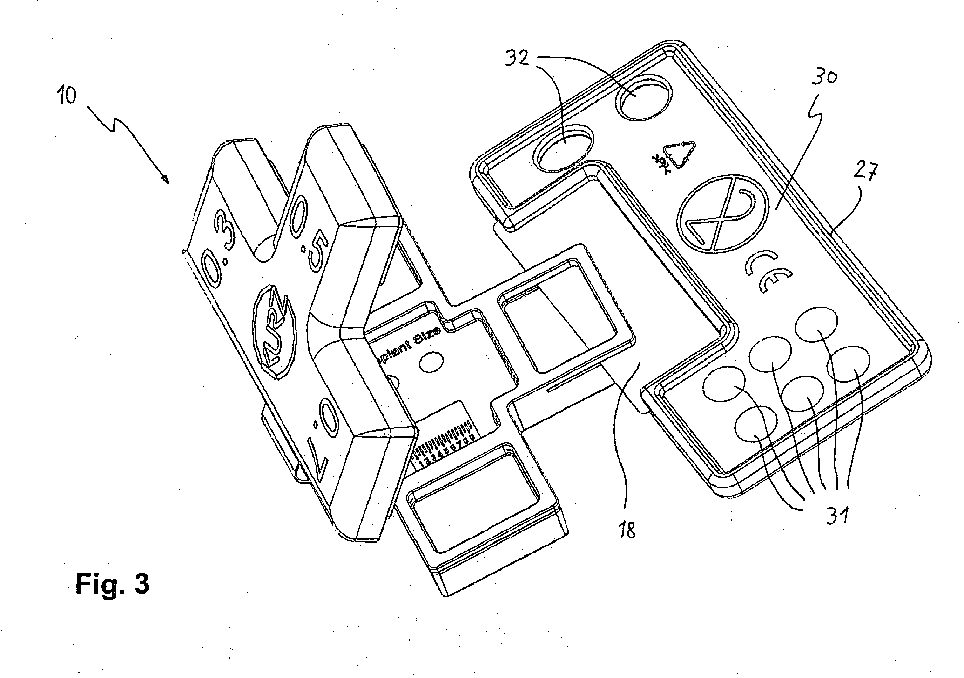

Die Schneidklinge 18 wird in der Regel eine Messerklinge aus Metall sein, insbesondere eine Rasiermesser-Klinge. Bei der in Fig. 3 gezeigten Ausführungsform ist die Schneidklinge 18 in einer einteiligen, vorzugsweise aus Kunststoff hergestellten **Messerhalterung 30** gehalten, die einen Schlitz zum Einschieben der Schneidklinge 18 aufweist. Bei in der Zeichnung nicht dargestellten Ausführungsformen der erfindungsgemäßen Schneidevorrichtung kann die Messerhalterung 30 auch anders, beispielsweise zweiteilig ausgeführt und zum Halten der Schneidklinge 18 klappbar gestaltet sein.

Die Messerhalterung 30 weist im gezeigten Ausführungsbeispiel mindestens eine Fläche auf, in der runde und ovale **Templates 31** unterschiedlichen Durchmessers eingearbeitet sind. Außerdem ist eine randseitig um die Messerhalterung 30 umlaufende **Umrandung 27** vorgesehen, die einen weiteren Arbeitsraum umgrenzt, und die Messerhalterung 30 weist **Durchgangsöffnungen 32** auf, die beispielsweise rund und/oder oval gestaltet sein können.

## Patentansprüche

1. Medizinische Schneidevorrichtung (10) zur Herstellung dünner Knorpelscheiben mit einem Vorrichtungskörper (11) und einem Deckel (12), wobei eine erste Halteeinrichtung vorgesehen ist, die einen ersten Abschnitt (11a) mit einer auf der Oberseite des Vorrichtungskörpers (11) angeordneten ersten Vertiefung (13a) aufweist, welche von einem ersten Begrenzungssteg (14a) ganz oder teilweise umrandet und durch einen auf der Oberseite des Deckels (12) angeordneten ersten Vorsprung (19a) verschließbar ist, und wobei der erste seitliche Begrenzungssteg (14a) einen von einer Stirnseite des ersten Abschnitts (11a) her geführten, mit einem vorgegebenen ersten Abstand parallel zur Grundfläche der ersten Vertiefung (13a) verlaufenden ersten Führungsschlitz (17a) zum Einschieben einer Schneidklinge (18) aufweist,
wobei die Schneidevorrichtung (10) aus einem sterilisierbaren Material hergestellt ist, **dadurch gekennzeichnet,**
**dass** mindestens eine zweite Halteeinrichtung vorgesehen ist, die einen zweiten Abschnitt (11b) mit einer auf der Oberseite des Vorrichtungskörpers (11) angeordneten zweiten Vertiefung (13b) aufweist, welche von einem zweiten Begrenzungssteg (14b) ganz oder teilweise umrandet und durch einen auf der Oberseite des Deckels (12) angeordneten zweiten Vorsprung (19b) verschließbar ist, wobei der zweite seitliche Begrenzungssteg (14b) einen von einer Stirnseite des zweiten Abschnitts (11b) her geführten, mit einem vorgegebenen zweiten Abstand parallel zur Grundfläche der zweiten Vertiefung (13b) verlaufenden zweiten Führungsschlitz (17b) zum Einschieben einer Schneidklinge aufweist.

2. Schneidvorrichtung nach Anspruch 1, **dadurch gekennzeichnet, dass** eine dritte Halteeinrichtung vorgesehen ist, die einen dritten Abschnitt (11c) mit einer auf der Oberseite des Vorrichtungskörpers (11) angeordneten dritten Vertiefung (13c) aufweist, welche von einem dritten Begrenzungssteg (14c) ganz oder teilweise umrandet und durch einen auf der Oberseite des Deckels (12) angeordneten dritten Vorsprung (19c) verschließbar ist, wobei der dritte Begrenzungssteg (14c) einen von einer Stirnseite des dritten Abschnitts (11c) her geführten, mit einem vorgegebenen dritten Abstand parallel zur Grundfläche der dritten Vertiefung (13c) verlaufenden dritten Führungsschlitz (17c) zum Einschieben einer Schneidklinge aufweist, und dass die drei Halteeinrichtungen relativ zueinander vorzugsweise in Kreuzform angeordnet sind.

3. Schneidevorrichtung nach einem der vorhergehenden Ansprüche, **dadurch gekennzeichnet, dass** die Schneidevorrichtung (10) in einem Spritzgussverfahren aus einem sterilisierbaren Kunststoff hergestellt ist, und dass der Anspritzpunkt für das Spritzgussverfahren auf der Soll-Biegelinie (23) angeordnet ist.

4. Schneidevorrichtung nach einem der vorhergehenden Ansprüche, **dadurch gekennzeichnet, dass** der Deckel (12) über eine Materialbrücke am Vorrichtungskörper (11) anhängt und ungefähr um 180° klappbar ist, und dass die Materialbrücke eine SollBiegelinie (23) oder Verengung aufweist, um welche der Deckel (12) gegenüber dem Vorrichtungskörper (11) klappbar ist.

5. Schneidvorrichtung nach einem der vorhergehenden Ansprüche, **dadurch gekennzeichnet, dass** an den Abschnitten (11a, 11b, 11c) des Vorrichtungskörpers (11) und/oder an den entsprechenden Abschnitten des Deckels (12), welche die Vorsprünge (19a, 19b, 19c) tragen, Kennzeichnungen (24a, 24b, 24c) angebracht sind, die den jeweiligen vorgegebenen Abstand des Führungsschlitzes (17a, 17b, 17c) zur Grundfläche der entsprechenden Vertiefung (13a, 13b, 13c) und somit die mit der jeweiligen Halteeinrichtung erzielbare Dicke der herzustellenden Knorpelscheibe angeben.

6. Schneidvorrichtung nach einem der vorhergehenden Ansprüche, **dadurch gekennzeichnet, dass** auf der den Vertiefungen (13a, 13b, 13c) gegenüberliegenden Unterseite des Vorrichtungskörpers (11) und/oder auf der den Vorsprüngen (19a, 19b, 19c) gegenüberliegenden Unterseite des Deckels (12) konvexe und/oder konkave Griffhilfen (25a, 25b, 25c) angebracht sind.

7. Schneidevorrichtung nach einem der vorhergehenden Ansprüche, **dadurch gekennzeichnet, dass** auf der den Vorsprüngen (19a, 19b, 19c) gegenüberliegenden Unterseite des Deckels (12) eine randseitig umlaufende Umrandung (26) vorgesehen ist.

8. Schneidvorrichtung nach einem der vorhergehenden Ansprüche, **dadurch gekennzeichnet, dass** auf der Oberseite des Vorrichtungskörpers (11) zwischen den Vertiefungen (13a, 13b, 13c) eine umlaufende Wandung (28) vorgesehen ist, die einen Arbeitsraum (29) umschließt.

9. Schneidevorrichtung nach einem der vorhergehenden Ansprüche, **dadurch gekennzeichnet, dass** in einer Fläche der Schneidevorrichtung (10), auf der die Vertiefungen (13a, 13b, 13c) tragenden Oberseite des Vorrichtungskörpers (11), im Arbeitsraum (29), und/oder auf der die Vorsprünge (19a, 19b, 19c) tragenden Oberseite des Deckels (12) runde und/oder ovale Templates (21) unterschiedlichen Durchmessers eingearbeitet sind.

10. Schneidevorrichtung nach einem der vorhergehenden Ansprüche, **dadurch gekennzeichnet, dass** in einer Fläche der Schneidevorrichtung (10), auf der die Vertiefungen (13a, 13b, 13c) tragenden Oberseite des Vorrichtungskörpers (11), im Arbeitsraum (29), und/oder auf der die Vorsprünge (19a, 19b, 19c) tragenden Oberseite des Deckels (12) eine Mess-Skala (22) eingearbeitet ist.

11. Schneidevorrichtung nach einem der vorhergehenden Ansprüche, **dadurch gekennzeichnet, dass** die Schneidklinge (18) eine Messerklinge aus Metall ist und in einer Messerhalterung (30) aus Kunststoff gehalten wird.

12. Schneidvorrichtung nach Anspruch 11, **dadurch gekennzeichnet, dass** die Messerhalterung (30) entweder einteilig ausgeführt ist und einen Schlitz zum Einschieben der Schneidklinge (18) aufweist, oder dass die Messerhalterung (30) zweiteilig ausgeführt und zum Halten der Schneidklinge (18) klappbar gestaltet ist.

13. Schneidvorrichtung nach Anspruch 11 oder 12, **dadurch gekennzeichnet, dass** die Messerhalterung (30) mindestens eine Fläche aufweist, in der runde und/oder ovale Templates (31) unterschiedlichen Durchmessers eingearbeitet sind.

14. Schneidvorrichtung nach einem der Ansprüche 11 bis 13, **dadurch gekennzeichnet, dass** eine randseitig um die Messerhalterung (30) umlaufende Umrandung (27) vorgesehen ist.

15. Schneidvorrichtung nach einem der Ansprüche 11 bis 14, **dadurch gekennzeichnet, dass** die Messerhalterung (30) mindestens eine Durchgangsöffnung (32) aufweist.

## Claims

1. Medical cutting device (10) for producing thin cartilage discs, comprising a device body (11) and a cover (12), provided with a first holding device having a first section (11a) with a first recess (13a) arranged on the top of the device body (11), wherein said recess is completely or partially bordered by a first delimiting web (14a) and can be closed by a first projection (19a) arranged on the top of the cover (12), wherein the first lateral delimiting web (14a) has a first guide slot (17a) leading from an end face of the first section (11a) and extending at a predetermined first distance parallel to the base of the first recess (13a) for the insertion of a cutting blade (18) and wherein the cutting device (10) is produced from a sterilisable material, **characterised by** at least a second holding device having a second section (11b) with a second recess (13b) arranged on the top of the device body (11), wherein said recess is completely or partially bordered by a second delimiting web (14b) and can be closed by a second projection (19b) arranged on the top of the cover (12) and wherein the second lateral delimiting web (14b) has a second guide slot (17b) leading from an end face of the second section (11b) and extending at a predetermined second distance parallel to the base of the second recess (13b) for the insertion of a cutting blade.

2. Cutting device according to claim 1, **characterised by** a third holding device having a third section (11c) with a third recess (13c) arranged on the top of the device body (11), wherein said recess is completely or partially bordered by a third delimiting web (14c) and can be closed by a third projection (19c) arranged on the top of the cover (12), wherein the third lateral delimiting web (14c) has a third guide slot (17c) leading from an end face of the third section (11c) and extending at a predetermined third distance parallel to the base of the third recess (13c) for the insertion of a cutting blade and wherein the three holding devices are preferably arranged in the shape of a cross relative to one another.

3. Cutting device according to one of the preceding claims, **characterised in that** the cutting device (10) is produced from a sterilisable plastic by injection moulding and that the injection point for the injection moulding process is arranged on the predetermined bending line (23).

4. Cutting device according to one of the preceding claims, **characterised in that** the cover (12) is attached to the device body (11) via a material bridge and can be hinged through approximately 180° and that the material bridge has a predetermined bending line (23) or narrowed portion about which the cover (12) can be hinged relative to the device body (11).

5. Cutting device according to one of the preceding claims, **characterised in that** markings (24a, 24b, 24c) are applied to the sections (11a, 11b, 11c) of the device body (11) and/or to the corresponding sections of the cover (12) bearing the projections (19a, 19b, 19c), said markings indicating the respective predetermined distance of the guide slot (17a, 17b, 17c) from the base of the corresponding recess (13a, 13b, 13c) and therefore the thickness of the cartilage disc to be produced that can be obtained with the respective holding device.

6. Cutting device according to one of the preceding claims, **characterised in that** convex and/or concave gripping aids (25a, 25b, 25c) are applied to the underside of the device body (11) opposite the recesses (13a, 13b, 13c) and/or to the underside of the cover (12) opposite the projections (19a, 19b, 19c).

7. Cutting device according to one of the preceding claims, **characterised by** a border (26) running around the edge of the underside of the cover (12) opposite the projections (19a, 19b, 19c).

8. Cutting device according to one of the preceding claims, **characterised by** a circumferential wall (28) enclosing a working space (29) on the top of the device body (11) between the recesses (13a, 13b, 13c).

9. Cutting device according to one of the preceding claims, **characterised in that** round and/or oval templates (21) of different diameters are incorporated into a surface of the cutting device (10) on the top of the device body (11) bearing the recesses (13a, 13b, 13c), in the working space (29), and/or on the top of the cover (12) bearing the projections (19a, 19b, 19c).

10. Cutting device according to one of the preceding claims, **characterised in that** a measuring scale (22) is incorporated into a surface of the cutting device (10) on the top of the device body (11) bearing the recesses (13a, 13b, 13c), in the working space (29), and/or on the top of the cover (12) bearing the projections (19a, 19b, 19c).

11. Cutting device according to one of the preceding claims, **characterised in that** the cutting blade (18) is a knife blade made of metal and is held in a knife holder (30) made of plastic.

12. Cutting device according to claim 11, **characterised in that** the knife holder (30) is either designed in one piece and has a slot for the insertion of the cutting blade (18) or the knife holder (30) is designed in two parts and has a hinged design for holding the cutting blade (18).

13. Cutting device according to claim 11 or claim 12, **characterised in that** the knife holder (30) has at least one surface into which round and/or oval templates (31) of different diameters are incorporated.

14. Cutting device according to one of claims 11 to 13, **characterised by** a border (27) running around the edge of the knife holder (30).

15. Cutting device according to one of claims 11 to 14, **characterised in that** the knife holder (30) has at least one through opening (32).

## Revendications

1. Dispositif de coupe (10) médical destiné à la fabrication de minces disques de cartilage, avec un corps de dispositif (11) et un couvercle (12), dans lequel il est prévu un premier dispositif de retenue qui présente un premier tronçon (11a) avec un premier creux (13a) qui est disposé sur le côté supérieur du corps de dispositif (11) et qui est bordé totalement ou partiellement par une première nervure de délimitation (14a) et qui peut être fermé par une première saillie (19a) disposée sur le côté supérieur du couvercle (12), et dans lequel la première nervure de délimitation (14a) latérale présente une première fente de guidage (17a) qui est conduite à partir d'un côté frontal du premier tronçon (11a) et placée, à une première distance prédéfinie, parallèlement à la surface de base du premier creux (13a), et est destinée à l'insertion d'une lame de coupe (18),
dans lequel le dispositif de coupe (10) est fabriqué en un matériau stérilisable,
**caractérisé en ce**
**qu'**il est prévu au moins un deuxième dispositif de retenue qui présente un deuxième tronçon (11 b) avec un deuxième creux (13b) qui est disposé sur le côté supérieur du corps de dispositif (11) et qui est bordé totalement ou partiellement par une deuxième nervure de délimitation (14b) et qui peut être fermé par une deuxième saillie (19b) disposée sur le côté supérieur du couvercle (12), dans lequel la deuxième nervure de délimitation (14b) latérale présente une deuxième fente de guidage (17b) qui est conduite à partir d'un côté frontal du deuxième tronçon (11 b) et placée, à une deuxième distance prédéfinie, parallèlement à la surface de base du deuxième creux (13b), et est destinée à l'insertion d'une lame de coupe.

2. Dispositif de coupe selon la revendication 1, **caractérisé en ce qu'**il est prévu un troisième dispositif de retenue qui présente un troisième tronçon (11 c) avec un troisième creux (13c) qui est disposé sur le côté supérieur du corps de dispositif (11) et qui est bordé totalement ou partiellement par une troisième nervure de délimitation (14c) et qui peut être fermé par une troisième saillie (19c) disposée sur le côté supérieur du couvercle (12), dans lequel la troisième nervure de délimitation (14c) présente une troisième fente de guidage (17c) qui est conduite à partir d'un côté frontal du troisième tronçon (11c) et placée, à une troisième distance prédéfinie, parallèlement à la surface de base du troisième creux (13c) et est destinée à l'insertion d'une lame de coupe, et **en ce que** les trois dispositifs de retenue sont disposés de préférence en croix les uns par rapport aux autres.

3. Dispositif de coupe selon l'une des revendications précédentes, **caractérisé en ce que** le dispositif de coupe (10) est fabriqué, selon un procédé de moulage par injection, en une matière plastique stérilisable, et **en ce que** le point d'injection pour le procédé de moulage par injection est disposé sur la ligne de flexion de consigne (23).

4. Dispositif de coupe selon l'une des revendications précédentes, **caractérisé en ce que** le couvercle (12) est suspendu au corps de dispositif (11) par le biais d'un pont de matière et peut être rabattu d'environ 180°, et **en ce que** le pont de matière présente une ligne de flexion de consigne (23) ou un rétrécissement autour de laquelle ou duquel le couvercle (12) peut être rabattu par rapport au corps de dispositif (11).

5. Dispositif de coupe selon l'une des revendications précédentes, **caractérisé en ce que**, sur les tronçons (11a, 11 b, 11 c) du corps de dispositif (11) et/ou sur les tronçons correspondants du couvercle (12) qui portent les saillies (19a, 19b, 19c), il est apposé des marquages (24a, 24b, 24c) qui indiquent la distance prédéfinie respective séparant la fente de guidage (17a, 17b, 17c) et la surface de base du creux (13a, 13b, 13c) correspondant et donc l'épaisseur du disque de cartilage à fabriquer qui peut être obtenue avec le dispositif de retenue respectif.

6. Dispositif de coupe selon l'une des revendications précédentes, **caractérisé en ce que** des aides de préhension (25a, 25b, 25c) convexes et/ou concaves sont mises en place sur le côté inférieur du corps de dispositif (11) opposé aux creux (13a, 13b, 13c) et/ou sur le côté inférieur du couvercle (12) opposé aux saillies (19a, 19b, 19c).

7. Dispositif de coupe selon l'une des revendications précédentes, **caractérisé en ce qu'**il est prévu une bordure (26) périphérique côté bord sur le côté inférieur du couvercle (12) opposé aux saillies (19a, 19b, 19c).

8. Dispositif de coupe selon l'une des revendications précédentes, **caractérisé en ce que**, sur le côté supérieur du corps de dispositif (11), entre les creux (13a, 13b, 13c), il est prévu une paroi (28) périphérique qui entoure un espace de travail (29).

9. Dispositif de coupe selon l'une des revendications précédentes, **caractérisé en ce que**, dans une surface du dispositif de coupe (10), sur le côté supérieur du corps de dispositif (11) portant les creux (13a, 13b, 13c), dans l'espace de travail (29), et/ou sur le côté supérieur du couvercle (12) portant les saillies (19a, 19b, 19c), des gabarits (21) ronds et/ou ovales de différents diamètres sont incorporés.

10. Dispositif de coupe selon l'une des revendications précédentes, **caractérisé en ce que**, dans une surface du dispositif de coupe (10), sur le côté supérieur du corps de dispositif (11) portant les creux (13a, 13b, 13c), dans l'espace de travail (29), et/ou sur le côté supérieur du couvercle (12) portant les saillies (19a, 19b, 19c), une échelle de mesure (22) est incorporée.

11. Dispositif de coupe selon l'une des revendications précédentes, **caractérisé en ce que** la lame de coupe (18) est une lame de couteau en métal et est retenue dans un élément de retenue de couteau (30) en matière plastique.

12. Dispositif de coupe selon la revendication 11, **caractérisé en ce que** soit l'élément de retenue de couteau (30) est réalisé en une seule partie et présente une fente pour l'insertion de la lame de coupe (18), soit **en ce que** l'élément de retenue de couteau (30) est réalisé en deux parties et est constitué de façon rabattable pour la retenue de la lame de coupe (18).

13. Dispositif de coupe selon la revendication 11 ou 12, **caractérisé en ce que** l'élément de retenue de couteau (30) présente au moins une surface dans laquelle des gabarits (31) ronds et/ou ovales de différents diamètres sont incorporés.

14. Dispositif de coupe selon l'une des revendications 11 à 13, **caractérisé en ce qu'**il est prévu une bordure (27) périphérique côté bord autour de l'élément de retenue de couteau (30).

15. Dispositif de coupe selon l'une des revendications 11 à 14, **caractérisé en ce que** l'élément de retenue de couteau (30) présente au moins une ouverture de passage (32).
